(19) Europäisches Patentamt
European Patent Office
Office européen des brevets

(11) **EP 2 393 445 B1**

(12) **EUROPÄISCHE PATENTSCHRIFT**

(45) Veröffentlichungstag und Bekanntmachung des Hinweises auf die Patenterteilung:
**20.11.2019 Patentblatt 2019/47**

(21) Anmeldenummer: **09799586.4**

(22) Anmeldetag: **09.12.2009**

(51) Int Cl.:
*A61B 1/04* *(2006.01)*　　　*A61B 1/045* *(2006.01)*
*A61B 34/00* *(2016.01)*

(86) Internationale Anmeldenummer:
**PCT/EP2009/066754**

(87) Internationale Veröffentlichungsnummer:
**WO 2010/088991 (12.08.2010 Gazette 2010/32)**

(54) **VORRICHTUNG ZUM ABLÖSEN EINER ENDOSKOPIEKAPSEL VON EINER OBERFLÄCHE EINER FLÜSSIGKEIT**

SYSTEM FOR SEPARATING AN ENDOSCOPY CAPSULE FROM A SURFACE OF A LIQUID

DISPOSITIF POUR LA SÉPARATION D'UNE CAPSULE ENDOSCOPIQUE D'UNE SURFACE D'UN LIQUIDE

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **05.02.2009 DE 102009007513**

(43) Veröffentlichungstag der Anmeldung:
**14.12.2011 Patentblatt 2011/50**

(73) Patentinhaber: **Siemens Healthcare GmbH**
**91052 Erlangen (DE)**

(72) Erfinder: **REINSCHKE, Johannes**
**90425 Nürnberg (DE)**

(56) Entgegenhaltungen:
**EP-A1- 1 972 253**　　　**EP-A1- 2 143 368**
**WO-A1-2009/001666**

- **Dr Med Wolfgang ET AL: "Internistische Gemeinschaftspraxis", , 16 August 2017 (2017-08-16), XP055399177, Retrieved from the Internet: URL:http://www.gastro-muc-solln.de/pdf/aufklaerung-kapsel-endoskopie.pdf [retrieved on 2017-08-17]**

**Beschreibung**

Die Erfindung betrifft eine Vorrichtung zum Ablösen einer magnetisch geführten Endoskopiekapsel von einer Wasseroberfläche mit dem Ziel, die Kapsel unter Verwendung möglichst geringer magnetischer Kräfte vollständig im Wasser unterzutauchen und dabei von der Wasseroberfläche abzulösen.

Die Verwendung von Endoskopiekapseln findet in der Medizin zur Diagnose oder zur Behandlung des Inneren eines Patienten immer breitere Anwendung. Eine Endoskopiekapsel kann u.a. medizinische Instrumente bspw. zur Biopsie oder zum Einbringen von Medikamenten in den Körper und/oder bildgebende Systeme wie Kameras beinhalten. Die Endoskopiekapsel weist ein fest mit der Kapsel verbundenes magnetisches Element mit einem magnetischen Dipolmoment auf, das bspw. von einem in der Kapsel fest installierten Permanentmagneten herrühren kann. Aufgrund des magnetischen Dipolmoments kann die Kapsel wie bspw. in der DE 10 2008 004 871 beschrieben mit Hilfe einer Manövriervorrichtung beliebig manövriert bzw. navigiert werden. Die DE 10 2008 004 871 beschreibt ein Magnetspulensystem bestehend aus mehreren Einzelspulen zur Navigation einer Endoskopiekapsel, einer Videokapsel oder einer sonstigen Sonde. Im Folgenden ist nur noch allgemein von einer "Endoskopiekapsel" oder kurz von einer "Kapsel" die Rede, wobei unter diesem Begriff die Endoskopiekapsel, die Videokapsel und die sonstigen Sonden zusammengefasst sind. In der Kapsel ist ein magnetisches Element, bspw. ein Permanent- oder Ferromagnet, fest installiert, so dass sie mit Hilfe des Magnetspulensystems beliebig manövrierbar ist. Das Magnetspulensystem erzeugt Magnetfeldkomponenten $B_x$, $B_y$, $B_z$ entlang der Achsen x, y, z eines kartesischen Koordinatensystems sowie magnetische Gradientenfelder, die eine berührungslose Führung der Endoskopiekapsel ermöglichen.

Hierbei wird ausgenutzt, dass sich - in Abwesenheit signifikanter mechanischer Gegenkräfte - das magnetische Element, d.h. ein Körper mit einem magnetischen Dipolmoment $\vec{m}$, parallel zur Richtung des magnetischen Feldes $\vec{B}$ bestehend aus den Magnetfeldkomponenten $B_x$, $B_y$, $B_z$ in Richtung der Achsen des kartesischen Koordinatensystems ausrichtet. Da das magnetische Element fest mit der Endoskopiekapsel verbunden ist, kann so die Orientierung der Kapsel beeinflusst werden. Zusätzlich wirkt, ausgelöst durch die magnetischen Gradientenfelder $\partial B_x / \partial x$ etc., eine Kraft $\vec{F} = \underline{\underline{G}} \cdot \vec{m}$ mit einer die Gradientenfelder umfassenden Gradientenmatrix $\underline{\underline{G}}$ auf das magnetische Dipolmoment $\vec{m}$ gemäß

$$\vec{F} = \underline{\underline{G}} \cdot \vec{m} = \begin{pmatrix} \partial B_x / \partial x & \partial B_x / \partial y & \partial B_x / \partial z \\ \partial B_y / \partial x & \partial B_y / \partial y & \partial B_y / \partial z \\ \partial B_z / \partial x & \partial B_z / \partial y & \partial B_z / \partial z \end{pmatrix} \cdot \vec{m}.$$

Die Gradientenmatrix $G$ ist aufgrund der Maxwell-Gleichungen rot $\vec{B} = 0$ und div $\vec{B} = 0$ symmetrisch und spurfrei, d.h. sie enthält mit $\partial B_x / \partial y$ ($= \partial B_y / \partial x$), $\partial B_x / \partial z$ ($= \partial B_z / \partial x$), $\partial B_y / \partial z$ ($= \partial B_z / \partial y$) und zweien der drei Diagonalelemente (bspw. $\partial B_x / \partial x$ und $\partial B_y / \partial y$) fünf unabhängige Gradientenfelder.

Durch eine gezielte Ansteuerung der Einzelspulen der Magnetspulenanordnung können das Magnetfeld $\vec{B}$ und eines oder mehrere der Gradientenfelder der Matrix $\underline{\underline{G}}$ beliebig eingestellt werden. Es ist damit zum einen möglich, das magnetische Element bzw. die Kapsel zu drehen und es somit beliebig in einem Arbeitsraum A innerhalb des Magnetspulensystems auszurichten. Zum anderen ist es möglich, eine Kraft $\vec{F}$ auf das magnetische Element auszuüben, um es zusätzlich zur Drehung translatorisch zu verschieben.

Zur näheren Erläuterung der Kapselnavigation mit Hilfe der diversen vom Magnetspulensystem erzeugten Felder sei insbesondere auf die DE 10 2008 004 871 verwiesen.

Eine spezielle Anwendung der magnetischen Kapselendoskopie ist das sog. Magen-Screening, das eine z.B. in der US 2007/0221233 A1 beschriebene Untersuchung des Magens umfasst. Beim Magen-Screening ist der Magen teilweise mit Wasser gefüllt und die Kapsel bzw. eine in der Kapsel integrierte Kamera, deren optische Achse üblicherweise in Richtung der Kapsellängsachse orientiert ist und die sich in der Regel an einem der Kapselenden in die Kapsel eingebaut ist, soll Fern- und Nahaufnahmen der Magenschleimhaut machen. Bei einer Fernaufnahme schwimmt die Kapsel in der Regel an der Wasseroberfläche, wobei eines der beiden im Allgemeinen halbkugelförmigen Kapselenden teilweise aus der Wasseroberfläche herausschaut. Für eine Nahaufnahme ist die Kapsel typischerweise vollständig ins Wasser eingetaucht. Für den Übergang von einer Fernaufnahme zu einer Nahaufnahme muss die Kapsel folglich von der Wasseroberfläche abgelöst werden, wozu aufgrund der Oberflächenspannung des Wassers eine magnetische Kraft in einer Größenordnung von etwa 2mN auf die Kapsel aufgebracht werden muss. Diese Ablösekraft ist deutlich größer als die magnetische Kraft, die benötigt wird, um die Kapsel langsam, d.h. im Falle des Magen-Screenings mit einer Geschwindigkeit von 0-5mm/sec, entweder in zwei Dimensionen an der Wasseroberfläche oder dreidimensional vollständig in Wasser eingetaucht zu bewegen. Typischerweise braucht man hier Kräfte in einer Größenordnung von etwa 0,2mN bis 0,3mN, wobei dies bei einer vertikalen Bewegung einer vollständig in Wasser eingetauchten Kapsel nur dann gilt, wenn die mittlere Dichte der Kapsel annähernd gleich der Dichte von Wasser ist.

Die ausgeübte magnetische Kraft ist proportional zu den Spulenströmen in den Einzelspulen des Magnetspulensystems. Um die zum vollständigen Untertauchen benötigte magnetische Kraft auf die Kapsel zu erzeugen, sind demnach Spulenströme bzw. Amperewindungszahlen in den Magnetspulen nötig, die deutlich über die für eine normale Navigation der Kapsel benötigten Ströme bzw. Amperewindungszahlen hinausgehen. Dementsprechend werden teure Leistungsverstärker, die die höheren Ströme erzeugen können, sowie entsprechend leistungsfähigere Kühlsysteme benötigt.

Eine Möglichkeit, den Einfluss der Oberflächenspannung zu reduzieren, besteht darin, die an der Wasseroberfläche schwimmende Kapsel derart zu schwenken, dass das aus der Wasseroberfläche herausschauende Ende mit Wasser benetzt wird. Nach dem Benetzen verhält sich die Kapsel wie eine vollständig eingetauchte Kapsel, die knapp unter der Wasseroberfläche schwebt, d.h. eine besondere Ablösekraft ist zum Absenken der Kapsel nicht mehr erforderlich. Beim Schwenken wird jedoch zwangsläufig der Blickwinkel der Kapsel bzw. der von der Kamera abgebildete Bereich geändert. Demzufolge verlässt der näher zu betrachtende Zielbereich auf der Magenschleimhaut, der bspw. mittels der Fernaufnahme identifiziert wurde, beim Schwenken in der Regel das Blickfeld der Kamera. Der Zielbereich muss dann erst wieder gesucht werden, bevor mit der näheren Untersuchung fortgefahren werden kann, was bei der vergleichsweise geringen Bildwiederholrate der Kapselkamera und unter den optischen Bedingungen im Magen recht zeitaufwändig werden kann.

Eine weitere Möglichkeit, um eine zum Ablösen der Kapsel von einer Flüssigkeitsoberfläche erforderliche Ablösekraft zu verringern, wird in der EP 2 143 368 A1 beschrieben, die Stand der Technik unter Artikel 54(3) EPÜ ist. Darin wird die Kapsel mittels eines wechselseitig rotierenden Magnetfelds in eine Schaukelbewegung versetzt, wobei eine Trägheitskraft erzeugt wird die zum Ablösen der Kapsel von der Flüssigkeitsoberfläche führt.

Weiterhin wird in der EP 1 972 253 A1 vorgeschlagen, lediglich den Neigungswinkel einer an einer Flüssigkeitsoberfläche schwimmenden Kapsel mittels eines Magnetspulensystems zu verändern, um dadurch eine Kamera der Kapsel auf einen bestimmten Zielbereich auszurichten. Allerdings ist damit eine nähere Untersuchung eines Zielbereichs nur bedingt möglich. Ein Untertauchen der Kapsel ist nicht vorgesehen.

Es ist daher die Aufgabe der vorliegenden Erfindung, eine Vorrichtung zum vollständigen Untertauchen einer Endoskopiekapsel unter eine Oberfläche einer Flüssigkeit anzugeben.

Diese Aufgabe wird durch die in dem unabhängigen Anspruch angegebene Erfindung gelöst. Vorteilhafte Ausgestaltungen ergeben sich aus den abhängigen Ansprüchen.

Eine erfindungsgemäße Vorrichtung zum vollständigen Untertauchen einer an einer Oberfläche einer Flüssigkeit in einem Patienten schwimmenden Endoskopiekapsel in die Flüssigkeit mit Ablösung der Endoskopiekapsel von der Oberfläche der Flüssigkeit umfasst eine Endoskopiekapsel, ein Magnetspulensystem zur Erzeugung eines Magnetfeldes und/oder eines magnetischen Gradientenfeldes zur magnetischen Führung der Endoskopiekapsel und einer Ansteuereinheit. Die Endoskopiekapsel weist ein fest integriertes magnetisches Element mit einem magnetischen Dipolmoment auf. In der Ansteuereinheit ist eine Navigationssoftware zur Steuerung der Bestromung der Einzelspulen des Magnetspulensystems und eine weitere Software zur Steuerung der Bestromung der Einzelspulen des Magnetspulensystems implementiert.

Bei der erfindungsgemäßen Lösung wird der zeitliche Verlauf der Kraft, die von dem Magnetspulensystem auf die Kapsel bzw. auf deren magnetisches Moment erzeugt wird, optimiert. Hierbei wird von einem Magnetspulensystem erfindungsgemäß ein kurzzeitiger Kraftverlauf mit zumindest einem Kraftpuls auf die Endoskopiekapsel erzeugt, wobei die Richtung der erzeugten Kraft im Wesentlichen senkrecht auf der Oberfläche der Flüssigkeit steht.

Der kurzzeitige Kraftverlauf wird in Abhängigkeit von die Endoskopiekapsel kennzeichnenden Parametern, insbesondere Geometrie, Oberflächenbeschaffenheit und Ausrichtung der Kapsellängsachse relativ zur Wasseroberfläche, sowie von die Flüssigkeit kennzeichnenden Parametern, insbesondere Temperatur, chemische Zusammensetzung, Reinheit und Viskosität, derart vorausberechnet, dass die Endoskopiekapsel nach Beendigung der Erzeugung des kurzzeitigen Kraftverlaufs vollständig untergetaucht ist und sich knapp unter der Oberfläche der Flüssigkeit in einem Schwebezustand befindet.

Erfindungsgemäß wird der kurzzeitige Kraftverlauf nach einem Auslösen durch einen Bediener automatisch erzeugt. Hiermit wird sichergestellt, dass insbesondere die Beträge der Kräfte des Kraftverlaufs korrekt eingestellt sind und dass die auf die Kapsel wirkenden Kräfte rechtzeitig abgeschaltet werden, so dass die Kapsel unter der Oberfläche zum Schweben kommt und nicht bspw. auf die gegenüberliegende Mageninnenwand aufschlägt.

In einer vorteilhaften Ausgestaltung ist der Kraftpuls durch ein weitestgehend kastenförmiges, ein rampenartiges, ein dreieckiges oder ein spline-artiges zeitliches Verhalten gekennzeichnet, wobei sich die Begriffe "kastenförmig", "rampenartig", "dreieckig" und "spline-artig" auf das zeitliche Verhalten der erzeugten Kraft beziehen. Die spezielle Formgebung der Profile, insbesondere die kastenförmigen Kraftpulse, haben den Vorteil, dass mit Ihnen nur vergleichsweise geringe magnetische Kräfte für das vollständige Untertauchen aufgebracht werden müssen.

In einer Ausgestaltung der erfindungsgemäßen Vorrichtung wird eine ungerade Anzahl von Kraftpulsen auf die Kapsel erzeugt, wobei die Richtung der Kraft jedes ungeraden Kraftpulses in die Flüssigkeit hinein weist, so dass ein zumindest teilweises Eintauchen der Endoskopiekapsel in die Flüssigkeit bewirkt wird, während die Richtung der Kraft jedes geraden Kraftpulses aus der Flüssigkeit heraus weist, so dass ein zumindest teilweises Auftauchen der Endoskopiekapsel aus der Flüssigkeit bewirkt wird.

In einer alternativen Ausgestaltung wird eine gerade Anzahl von Kraftpulsen erzeugt, wobei die Richtung der Kraft jedes ungeraden Kraftpulses aus der Flüssigkeit heraus weist, so dass ein zumindest teilweises Auftauchen der Endoskopiekapsel aus der Flüssigkeit bewirkt wird, während die Richtung der Kraft jedes geraden Kraftpulses in die Flüssigkeit hinein weist, so dass ein zumindest teilweises Eintauchen der Endoskopiekapsel in die Flüssigkeit bewirkt wird.

Sowohl die Ausgestaltung mit einer ungeraden Anzahl von Kraftpulsen als auch die Alternative mit einer geraden Anzahl von Kraftpulsen erweist sich als vorteilhaft, da der zum Untertauchen benötigte Betrag der Amplituden der Kraftpulse gegenüber nur einem Kraftpuls deutlich geringer ausfällt.

Vorteilhafterweise werden die Beträge der Amplituden der Kraftpulse und das zeitliche Verhalten der Kraftpulse auf eine Schwingresonanz eines Feder-Masse-Systems abgestimmt, wobei der Feder-Anteil des Feder-Masse-Systems durch die Oberflächenspannung der die Kapsel umgebenden Flüssigkeit bestimmt wird und der Masse-Anteil aus der Masse der Endoskopiekapsel und der Masse eines die Endoskopiekapsel umgebenden Anteils der Flüssigkeit, der mit der Endoskopiekapsel mitbewegt wird, besteht. Hierdurch wird erreicht, dass die Kraftpulse optimiert werden können, so dass zum einen ein vollständiges Untertauchen garantiert ist und zum anderen lediglich die minimal benötigten Ströme zur Versorgung der Spulen des Magnetspulensystems aufgebracht werden müssen, dass eine Überdimensionierung des Systems also überflüssig ist.

Weiterhin wird in einer besonderen Ausführungsform automatisch, insbesondere durch ein Kapsel-Bewegungsmodell, ermittelt, ob sich die Endoskopiekapsel gerade an der Oberfläche der Flüssigkeit befindet oder ob die Endoskopiekapsel vollständig untergetaucht ist. Auch kann der Bediener manuell vorgeben, ob sich die Endoskopiekapsel gerade an der Oberfläche der Flüssigkeit befindet oder ob die Endoskopiekapsel vollständig untergetaucht ist. Der kurzzeitige Kraftverlauf kann nicht erzeugt werden, wenn die Endoskopiekapsel bereits vollständig untergetaucht ist. Diese Ausführungsform bietet den Vorteil erhöhter Sicherheit. Außerdem ist die Applikation des kurzzeitigen Kraftverlaufes, der auf die mechanische Resonanz der Kapsel an der Flüssigkeitsoberfläche abgestimmt ist, eben nur dann sinnvoll, wenn sich die Kapsel an der Flüssigkeitsoberfläche befindet. Andernfalls kann dieser Kraftverlauf eine unkontrollierte bis erratische Kapselbewegung verursachen. Es wäre nicht auszuschließen, dass die Kapsel ungewollt bspw. auf der Mageninnenwand aufschlägt und/oder unkontrolliert die mit Hilfe der Fernaufnahme ausgewählte Kapselposition verlässt.

Vorteilhafterweise umfasst die weitere Software ein Kapsel-Bewegungsmodell, mit dem ausgehend von einem definierten Ausgangszustand der Endoskopiekapsel und in Abhängigkeit von mittels des Magnetspulensystems auf die Endoskopiekapsel erzeugten magnetischen Kräften ermittelbar ist, ob sich die Endoskopiekapsel gerade an der Oberfläche der Flüssigkeit befindet oder ob die Endoskopiekapsel vollständig untergetaucht ist.

Alternativ oder zusätzlich weist die Ansteuereinheit eine Eingabeeinrichtung auf, mit der ein Bediener manuell vorgeben kann, ob sich die Endoskopiekapsel gerade an der Oberfläche der Flüssigkeit befindet oder ob die Endoskopiekapsel vollständig untergetaucht ist.

Das vollständige Untertauchen zeichnet sich dadurch aus, dass sich die Wasseroberfläche, unter die die Kapsel getaucht werden soll, über der Kapsel schließt.

Weitere Vorteile, Merkmale und Einzelheiten der Erfindung ergeben sich aus dem im Folgenden beschriebenen Ausführungsbeispiel sowie anhand der Zeichnungen.

Dabei zeigen:

Figur 1A     einen Querschnitt in nicht maßstabsgetreuer Ansicht durch den Magen eines in einer Magnetspulenanordnung gelagerten Patienten,

Figur 1B     eine Ansicht einer geeigneten Magnetspulenanordnung,

Figur 2A     das Ergebnis einer Simulation zur Abhängigkeit der Kapselposition von einem ersten Kraftverlauf in Form eines kurzen rampenförmigen Kraftpulses,

Figur 2B     das Ergebnis einer Simulation zur Abhängigkeit der Kapselposition von einem zweiten Kraftverlauf mit gleicher Länge und Form wie der erste Kraftverlauf, aber mit etwas steilerer Rampe,

Figur 2C     das Ergebnis einer Simulation zur Abhängigkeit der Kapselposition von einem dritten Kraftverlauf mit einem kastenförmigen Kraftpuls,

Figur 2D     das Ergebnis einer Simulation zur Abhängigkeit der Kapselposition von einem vierten Kraftverlauf mit drei kastenförmigen Kraftpulsen.

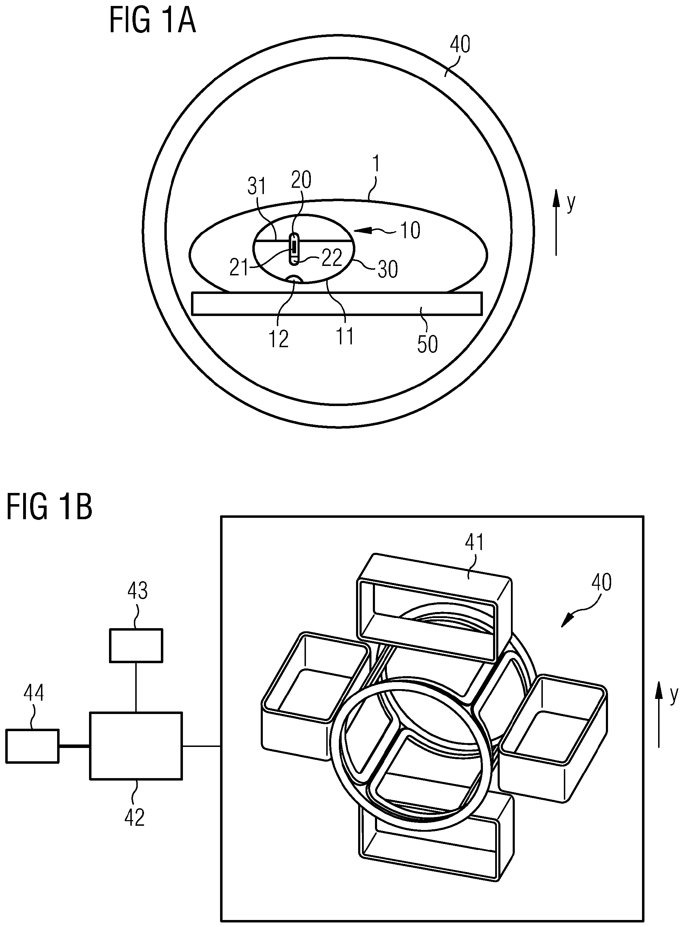

Die Figur 1A zeigt in einer nicht maßstabsgetreuen Darstellung den Magen 10 eines Patienten 1. Im Magen 10 befindet sich zur Durchführung eines Magen-Screenings eine Endoskopiekapsel 20 mit einem Permanentmagneten 21. Der Magen 10 ist zum Teil mit Wasser 30 gefüllt, und die Endoskopiekapsel 20 schwimmt an der Wasseroberfläche 31. Die Längsachse der Endoskopiekapsel 20 ist in Figur 1A in y-Richtung orientiert. Die Längsachse der Endoskopiekapsel 20 und die y-Richtung müssen nicht übereinstimmen, sondern können in einem Winkel von bis zu etwa 70° zueinander stehen. Die y-Richtung ist im Folgenden als diejenige Richtung definiert, die senkrecht zur Wasseroberfläche 31 steht. Dabei ist die positive y-Richtung aus dem Wasser 30 hinaus orientiert. Eine Bewegung in der negativen y-Richtung bedeutet demzufolge ein Ein- bzw. Untertauchen der Endoskopiekapsel 20, während eine Bewegung der Kapsel 20 in positive y-Richtung einem Auftauchen gleichkommt.

Der Patient 1 liegt zur Untersuchung auf einer Patientenliege 50 und befindet sich innerhalb eines Magnetspulensystems 40 mit einer Vielzahl von Einzelspulen 41, von denen der Übersichtlichkeit wegen in der Figur 1B nur eine mit einem Bezugszeichen versehen ist. Das Magnetspulensystem 40 umfasst auch die nicht dargestellten Leistungsverstärker. Als Magnetspulensystem 40 kann ein Spulensystem zum Einsatz kommen, wie es bspw. in der DE 10 2008 004 871 oder in der

DE 103 40 925 B3 beschrieben wird. Eine mögliche Ausführungsform des Magnetspulensystems 40 mit zehn Einzelspulen 41, die besonders geeignet ist, um die Endoskopiekapsel 20 im Magen 10 des Patienten 1 zu navigieren, ist exemplarisch in der Figur 1B dargestellt.

Das Magnetspulensystem 40 wird verwendet, um über die Erzeugung von Komponenten $B_x$, $B_y$, $B_z$ eines Magnetfeldes $\vec{B}$ und/oder von Gradientenfeldern der Gradientenmatrix $\underline{\underline{G}}$ Drehmomente und/oder Kräfte F_mag auf das magnetische Element 21 der Endoskopiekapsel 20 zu erzeugen. Da das magnetische Element 21 fest mit der Kapsel 20 verbunden ist, ergibt sich, dass die erzeugten Kräfte auch direkt auf die Endoskopiekapsel 20 wirken.

Mit den im Folgenden mit F_mag u.ä. bezeichneten magnetischen Kräften sind also diejenigen Kräfte gemeint, die in Wechselwirkung zwischen dem magnetischen Element 21 der Endoskopiekapsel 20 und den durch das Magnetspulensystem 40 erzeugten Gradientenfeldern der Gradientenmatrix $\underline{G}$ auf die Endoskopiekapsel 20 wirken.

Wie bereits erwähnt wird zur genauen Erläuterung der Wechselwirkung zwischen dem Magnetspulensystem 40 und dem Permanentmagneten 21 bzw. dessen magnetischen Dipolmoments auf die DE 10 2008 004 871 verwiesen.

Zur Steuerung des Magnetspulensystems 40 ist eine Ansteuereinheit 42 vorgesehen, in der mit Hilfe einer entsprechenden Navigationssoftware die Bestromung der Einzelspulen 41 zur Erzeugung der Magnet- und Gradientenfelder gesteuert wird. Bspw. kann ein Bediener des Magnetspulensystems 40 manuell mit Hilfe einer Bedieneinheit 43, bspw. ein Joystick, die Erzeugung der Magnet- und Gradientenfelder derart beeinflussen, dass je nach Richtung der Auslenkung des Joysticks 43 Felder in bestimmten Raumrichtungen erzeugt werden, wobei der Betrag des erzeugten Feldes von der Amplitude der Auslenkung des Joysticks 43 abhängen kann.

In die Endoskopiekapsel 20 ist eine Kamera 22 integriert, deren optische Achse in Richtung der Längsachse der Kapsel 20 orientiert ist. Mit der Kamera 22 werden zunächst Fernaufnahmen der Magenschleimhaut 11 gemacht, wobei eine Unregelmäßigkeit 12, bspw. ein Ulcus, entdeckt wird. Um die Unregelmäßigkeit 12 genauer untersuchen zu können, müssen Nahaufnahmen gemacht werden, wofür die Endoskopiekapsel in der negativen y-Richtung näher an die Unregelmäßigkeit 12 heran bewegt und letztendlich vollständig untergetaucht werden muss.

Grundsätzlich ist zum vollständigen Untertauchen und zum hiermit verbundenen Ablösen der Endoskopiekapsel 20 von der Wasseroberfläche 31 zu bemerken, dass der Ablösevorgang als dynamischer Prozess zu betrachten ist. Davon ausgehend, dass die Kapsel 20 mit der Masse M zunächst an der Wasseroberfläche 31 schwimmt und nun mit dem Magnetspulensystem 40 eine magnetische Kraft F_mag auf die Kapsel 20 in negative y-Richtung ausgeübt wird, so hängt die Bewegung der Kapsel 20 stark vom zeitlichen Verlauf der ausgeübten Kraft F_mag ab. Ist F_mag zu gering, werden die Kapsel 20 und die sie umgebende Wasseroberfläche 31 zwar in Stück nach unten abgesenkt, aber eine Ablösung der Kapsel 20 von der Oberfläche 31 und ein vollständiges Untertauchen finden nicht statt. Vielmehr schwingen die Kapsel 20 und die Wasseroberfläche 31 in der Umgebung der Kapsel 20 wieder zurück nach oben. Bei unzureichender Krafteinwirkung bewegt sich die Kapsel 20 zusammen mit dem umgebenden Wasser wie ein gedämpfter Oszillator bzw. wie ein gedämpftes Feder-Masse-System mit einer Federkonstanten k_surf, einer Dämpfung k_fric + k_fricW, wobei k_fric der Reibungskoeffizient der Kapsel und k_fricW der Reibungskoeffizient des umgebenden Wassers ist, und einer Masse M+m, wobei M die Masse der Kapsel und m die Masse des mitbewegten Wassers ist. Der Feder-Anteil des Feder-Masse-Systems wird durch die Oberflächenspannung der die Kapsel 20 umgebenden Flüssigkeit bestimmt, während der Masse-Anteil aus der Masse M der Endoskopiekapsel 20 und der Masse m des die Endoskopiekapsel 20 umgebenden Anteils der Flüssigkeit, der mit der Endoskopiekapsel mitbewegt wird, besteht.

Erst wenn die Auslenkung von Kapsel 20 und Wasseroberfläche nach unten einen kritischen Wert überschreitet, ist der Energieeintrag in die Wasseroberfläche groß genug, dass sich nach Ablösen des Wasserrandes von der Kapsel 20 die Wasseroberfläche über der Kapsel schließen kann. Nach diesem Ablösevorgang bewegt sich die Kapsel 20 im Wasser wie ein einfacher Körper mit einer Masse M und einem hydrodynamischen Reibungskoeffizienten k_fric.

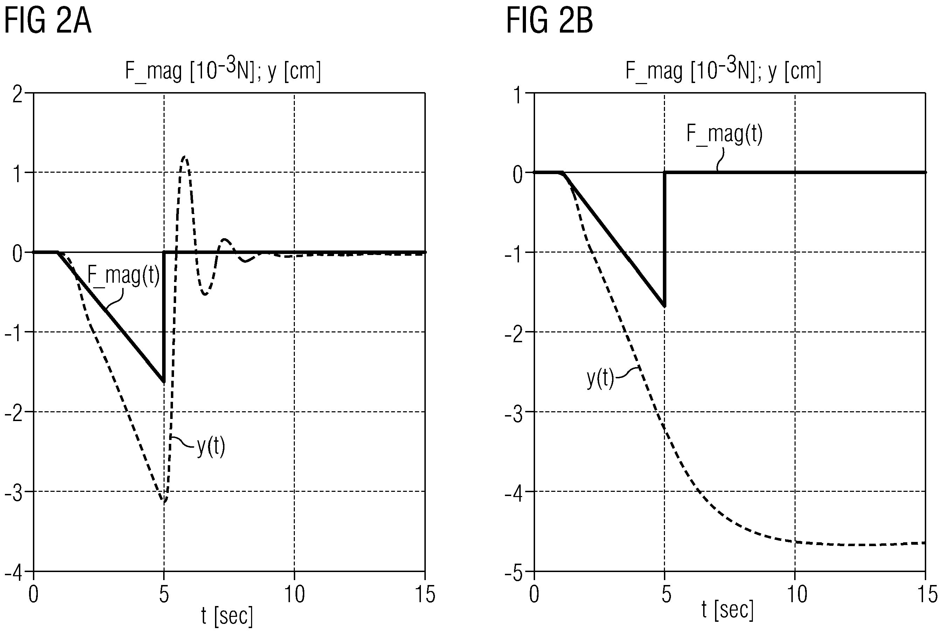

Die Figur 2A zeigt das Ergebnis einer Simulation der Abhängigkeit der Kapselposition y(t) in y-Richtung von der auf die Kapsel ausgeübten Kraft F_mag. Der Kraftverlauf F_mag(t) bzw. der zeitliche Verlauf F_mag(t) der Kraft F_mag ist dadurch charakterisiert, dass der Betrag |F_mag(t)| der Kraft allmählich bis zu einem Maximum von 1,6mN rampenartig ansteigt und anschließend auf Null abfällt. Die Kraft F_mag wirkt dabei in negative y-Richtung. Ebenfalls in Abhängigkeit von der Zeit t ist in der Figur 2A die Kapselposition y(t) in der y-Richtung dargestellt. Die Simulation zeigt den Fall, in dem die Kraft F_mag nicht ausreicht, die Kapsel 20 von der Wasseroberfläche abzulösen. Die Kapsel 20 wird zwar eingetaucht, nach Abschalten der Kraft F_mag führt sie jedoch eine oszillierende Bewegung in y-Richtung durch, um schließlich wieder an der Wasseroberfläche 31 zur Ruhe zu kommen.

Die Figur 2B zeigt einen im Wesentlichen gleichen zeitlichen Verlauf der Kraft F_mag wie in der Figur 2A. Der rampenartige Anstieg des Kraftbetrags ist jedoch etwas steiler als in der Figur 2A, so dass auch das Maximum des Betrags der Kraft F_mag etwas größer als im Fall der Figur 2A ist, d.h. größer als 1,6mN. In diesem Fall kommt es zu einem Ablösen der Kapsel 20 von der Wasseroberfläche 31 und zu einem vollständigen Untertauchen, d.h. die Wasseroberfläche schließt sich über der Kapsel. Die Kapsel 20 taucht demnach unter und kommt aufgrund der Reibung in einer bestimmten Tiefe zum Stillstand.

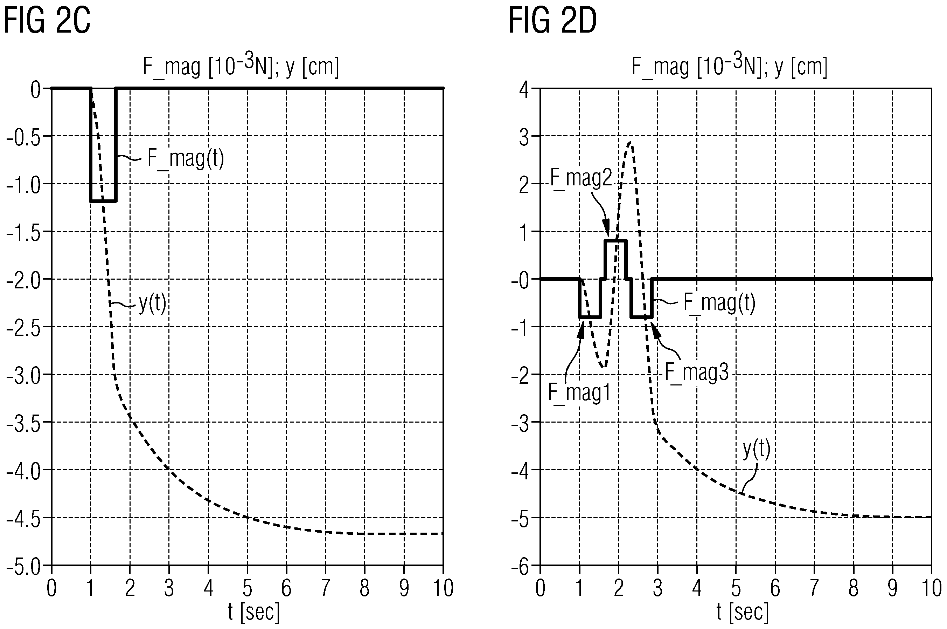

In der Figur 2C ist das Ergebnis einer weiteren Simulation dargestellt, bei der der zeitliche Verlauf der erzeugten magnetischen Kraft F_mag verändert wurde. Anstelle des rampenartigen Anstiegs wird hier ein Kraftpuls F_mag(t) mit einem kastenförmigen Profil bzw. einem kastenförmigen zeitlichen Verlauf erzeugt, der in die negative y-Richtung wirkt.

Bei einem ideal kastenförmigen Kraftpuls steigt die erzeugte Kraft zu einem ersten Zeitpunkt sprungartig von einem ersten Betrag, der in der Regel auf dem Nullniveau liegt, auf einen zweiten Betrag an, der zweite Betrag wird dann für eine gewisse Zeitspanne beibehalten, und zu einem zweiten Zeitpunkt fällt die Kraft wiederum sprungartig auf den ersten Betrag zurück.

Das zeitliche Verhalten der Kraft hängt jedoch direkt vom zeitlichen Verhalten der die Einzelspulen des Magnetspulensystems 40 durchfließenden Ströme ab. Das zeitliche Verhalten dieser Spulenströme kann aufgrund der Induktivität der Spulen sowie aufgrund der technisch bedingt begrenzten Spannungen der Leistungsverstärker (nicht dargestellt), die die Spulen versorgen, nur näherungsweise kastenförmig sein, womit auch das zeitliche Verhalten der erzeugten Kraft nur näherungsweise kastenförmig sein kann. Dabei zeichnet sich ein "näherungsweise" kastenförmiger Kraftpuls dadurch aus, dass

a) der Anstieg der Kraft von dem ersten auf den zweiten Betrag bzw. der entsprechende Anstieg des Spulenstroms in einer möglichst kurzen Zeit erfolgt und
b) ein Kraftplateau bzw. ein entsprechendes Stromplateau auf dem Niveau des zweiten Betrags für eine Zeitspanne, die ein Vielfaches der Anstiegszeit ist, gehalten wird.

Wenn im Folgenden die Rede von einem kastenförmigen Kraft- oder Strompuls die Rede ist, so ist jeweils ein näherungsweise kastenförmiger Puls gemeint. Ansonsten wird von einem idealen kastenförmigen Puls gesprochen. Der näherungsweise kastenförmige Puls wird technisch typischerweise so realisiert, dass unter Berücksichtigung der gegebenen technischen Voraussetzungen die minimal möglichen Anstiegszeiten vom ersten auf den zweiten Kraftbetrag realisiert werden. Die technischen Gegebenheiten umfassen hierbei die Leistungsparameter der Leistungsverstärker und die Eigenschaften der Magnetspulen, insbesondere deren Induktivität.

Für die Simulationen, die den Diagrammen der Figuren 2C und 2D zu Grunde liegen, wurden ideal kastenförmige Kraftpulse herangezogen.

Die Figur 2C zeigt, dass ein vollständiges Untertauchen und Ablösen von der Wasseroberfläche mit einem kastenförmigen Kraftpuls F_mag bereits mit einem Kraftbetrag von |F_mag|=1,2mN möglich ist. Auch in diesem Fall taucht die Kapsel 20 vollständig unter und kommt aufgrund der Reibung in einer bestimmten Tiefe zum Stillstand.

Eine weitere Reduzierung der benötigten Kraftamplitude lässt sich dadurch erreichen, dass mehrere kastenförmige Kraftpulse nacheinander erzeugt werden, wobei insbesondere eine ungerade Anzahl von Kraftpulsen vorteilhaft ist. Die Kraftpulse sind in ihrem zeitlichen Verlauf auf eine Schwingresonanz des bereits erwähnten gedämpften Feder-Masse-Systems bestehend aus der Kapsel und dem umgebenden Wasser vor der Ablösung der Kapsel von der Wasseroberfläche abgestimmt. Insbesondere werden dabei die Beträge der Amplituden der Kraftpulse und das zeitliche Verhalten der Kraftpulse auf die Schwingresonanz abgestimmt.

Dabei weist die Richtung der Kraft jedes ungeraden Kraftpulses, d.h. die Richtung der Kraft des ersten, dritten, fünften etc. Kraftpulses, in die negative y-Richtung bzw. in die Flüssigkeit hinein. Die ungeraden Kraftpulse bewirken also ein Eintauchen der Endoskopiekapsel in die Flüssigkeit. Die Richtung der Kraft jedes geraden Kraftpulses, d.h. die Richtung der Kraft des zweiten, vierten etc. Kraftpulses, weist dagegen aus der Flüssigkeit heraus. Die geraden Kraftpulse bewirken demzufolge ein Auftauchen der Endoskopiekapsel aus der Flüssigkeit.

Die Figur 2D zeigt in diesem Zusammenhang das Ergebnis einer Simulation mit einem zeitlichen Kraftverlauf, bei dem drei aufeinanderfolgende Kraftpulse F_mag1, F_mag2 und F_mag3 erzeugt wurden. Die Richtungen der ungeraden Kraftpulse F_mag1 und F_mag3 weisen in die Flüssigkeit hinein, während der gerade Kraftpuls F_mag2 in die entgegengesetzte, positive y-Richtung Richtung wirkt. Ebenfalls dargestellt ist der zeitliche Verlauf y(t) der Kapselposition in y-Richtung. Der erste Kraftpuls F_mag1 bewirkt, dass die Endoskopiekapsel in die Flüssigkeit eintaucht, aber noch nicht vollständig untertaucht. Der zweite Kraftpuls F_mag2 wirkt in entgegengesetzter Richtung und bewirkt ein Auftauchen der Kapsel aus der Flüssigkeit, während der dritte Kraftpuls F_mag3 schließlich das vollständige Untertauchen der Endoskopiekapsel unter der Oberfläche der Flüssigkeit erreicht. Da nach dem Ende des dritten Kraftpulses F_mag3 keine Kraft mehr auf die Kapsel ausgeübt wird, sinkt diese nicht unkontrolliert weiter, sondern wird durch die Reibung mit der Flüssigkeit abgebremst und kommt schließlich knapp unter der Oberfläche zum Stillstand.

Die Plateaus der Kraftpulse F_mag1 bis F_mag3 bzw. der Kraftbeträge |F_mag1| bis |F_mag3|, die benötigt wurden, um ein vollständiges Untertauchen der Endoskopiekapsel zu ermöglichen, lagen hier bei nur 0,8mN.

Die Simulationen zeigen, dass eine rampenartige, relativ langsame Krafterhöhung, wie sie in den Figuren 2A und 2B dargestellt ist, zu einem vergleichsweise hohen Kraftbedarf von |F_mag|>1,6mN im Maximum von F_mag(t) führt. Mit einem einzelnen kastenförmigen Kraftpuls wie in der Figur 2C kann unter ansonsten gleichen Bedingungen eine Ablösung der Kapsel von der Wasseroberfläche und ein vollständiges Untertauchen bereits erreicht werden, wenn der Betrag des Kraftpulses bei mindestens 1,2mN liegt. Bei einer Sequenz von drei aufeinanderfolgenden Kraftpulsen genügt bereits ein Betrag von nur jeweils 0,8mN.

In einer realistischen Anwendung ist darauf zu achten, dass die erzeugte Kraft F_mag zwar gerade zum Ablösen der Kapsel 20 von der Wasseroberfläche 31 ausreicht, dass aber eine darüber hinaus gehende, ggf. unkontrollierte Abwärtsbewegung der Kapsel 20 vermieden werden soll. Die Endoskopiekapsel 20 soll idealerweise unmittelbar nach der Ablösung unterhalb der Wasseroberfläche 31 zum Schweben kommen und insbesondere nicht etwa auf die unterhalb der Kapsel 20 liegende Magenschleimhaut 11 aufschlagen. Eine solche Vorgabe ist bei einer manuellen Steuerung der Kapsel 20, bei der bspw. mit Hilfe eines von Hand bedienten Joysticks Vorgaben für die Bestromung der Einzelspulen des Magnetspulensystems 40 zur Erzeugung der gewünschten Magnet- und Gradientenfelder erzeugt werden, nur schwer realisierbar, da die magnetische Kraft F_mag zur Ablösung der Kapsel 20 typischerweise mit einer zeitlichen Genauigkeit von etwa 0,lsec abgeschaltet werden muss, um eine unkontrollierte Bewegung unterhalb der Wasseroberfläche 31 zu vermeiden. Ferner ist der benötigte Kraftverlauf davon abhängig, unter welchem Winkel die Kapsellängsachse zur y-Richtung vor und während der Ablösung von der Flüssigkeitsoberfläche steht. Vorteilhafterweise wird daher der Prozess des Untertauchens der Kapsel 20, d.h. das Berechnen und das Erzeugen des Kraftverlaufs F_mag oder der Kraftverläufe F_mag1, F_mag2, F_mag3, automatisch vollzogen, ohne dass der Bediener manipulierend eingreifen muss. Dieser automatische Prozess ist in eine Steuereinheit implementiert, die mit dem Magnetspulensystem 40 verbunden ist und so die benötigten Kraftverläufe erzeugen kann. Dabei bietet es sich insbesondere an, als Steuereinheit die bereits vorhandene Ansteuereinheit 42 des Magnetspulensystems 40 zu verwenden und den automatischen Prozess in die Ansteuereinheit 42 zu implementieren. Effektiv beschränkt sich das notwendige Eingreifen des Bedieners darauf, dass er den automatischen Prozess des Untertauchens auslöst, bspw. indem er einen entsprechenden Tastschalter o.ä. der Ansteuereinheit 42 betätigt.

Nach der Betätigung des Tastschalters und davon ausgehend, dass sich die Endoskopiekapsel 20 zu diesem Zeitpunkt an der Wasseroberfläche 31 befindet, wird durch die Ansteuereinheit 42 ein kurzzeitiger Kraftverlauf F_mag(t) erzeugt, der bewirkt, dass die Kapsel 20 vollständig unter die Wasseroberfläche 31 untertaucht und anschließend nahe unter der Wasseroberfläche 31 in einem Schwebezustand verbleibt. An diesen kurzzeitigen Kraftverlauf, der einen der in den Figuren 2B bis 2D gezeigten Kraftverläufe umfassen kann, kann sich dann ein Kraftverlauf entsprechend einer vom Bediener gewünschten Soll-Kraft anschließen, die durch den Bediener mit Hilfe der Bedieneinheit 43 wie oben beschrieben vorgegeben wird und die die gewünschte Navigation der Kapsel 20 unter Wasser ermöglicht. Bspw. würde es sich im Falle der Untersuchung gemäß Figur 1A anbieten, die Kapsel 20 weiter in y-Richtung zu bewegen, um eine Nahaufnahme der Unregelmäßigkeit 12 erzeugen zu können.

Der kurzzeitige Kraftverlauf zeichnet sich gegenüber der normalen Navigation der Kapsel u.a. dadurch aus, dass höhere magnetische Kräfte erzeugt werden können, wobei magnetische Kräfte von maximal 0,2mN bis 0,3 mN typischerweise genügen. Dadurch wird verhindert, dass bei der normalen, manuellen Navigation die Kapsel mit zu starken Kräften beaufschlagt wird und sich zu schnell bewegt.

Zur Steuerung des Magnetspulensystems 40 ist bereits die Ansteuereinheit 42 eingeführt worden. Die Ansteuereinheit 42 kann zusätzlich dazu genutzt werden, zu ermitteln, ob sich die Endoskopiekapsel 20 gerade an der Wasseroberfläche 31 befindet oder ob die Kapsel 20 vollständig untergetaucht ist. Hierzu ist in der Ansteuereinheit 42 eine entsprechende Software implementiert.

In der Software in der Ansteuereinheit 42 ist bspw. ein einfaches Bewegungsmodell der Kapsel 20 hinterlegt, mit dem sich, davon ausgehend, dass eine Ausgangsposition der Kapsel 20 bekannt ist, die Kapselposition in Abhängigkeit von auf die Kapsel 20 ausgeübten Kräften berechnen lässt. Mit Hilfe eines definierten Ausgangszustandes der Kapsel 20, der eine Ausgangsposition und -ausrichtung der Kapsel 20 umfasst, sowie unter Verwendung der bspw. vom Joystick 43 eingehenden Steuerbefehle, d.h. Richtung und Amplitude der Auslenkung des Joysticks 43, ermittelt das Bewegungsmodell die ungefähre y-Position der Kapsel relativ zur Ausgangsposition und insbesondere, ob die Kapsel 20 an der Wasseroberfläche 31 schwimmt oder nicht. Dabei wird ab einem Anfangszeitpunkt der gesamte zeitliche Verlauf der auf die Kapsel wirkenden Magnetkräfte berücksichtigt. Typischerweise wird zu Beginn der Screening-Prozedur die Kapsel an der Wasseroberfläche sein bzw. dorthin magnetisch bewegt. Vorteilhaft für die Realisierung eines solchen Bewegungsmodells ist es, wenn für den Fall, dass vom Bediener über einen gewissen Zeitraum keine Eingabe über den Joystick 43 oder über eine andere Bedieneinheit wie bspw. eine Tastatur erfolgt ist, automatisch in einen Betriebsmodus der Ansteuereinheit 42 umgeschaltet wird, bei dem die Endoskopiekapsel 20 an die Wasseroberfläche 31 gezogen wird. Es kann dann sicher davon ausgegangen werden, dass sich die Kapsel 20 an der Oberfläche 31 befindet, so dass ein definierter Zustand "Endoskopiekapsel schwimmt an der Wasseroberfläche" erreicht ist.

Alternativ oder zusätzlich wird dem Bediener über eine graphische Bedienoberfläche (GUI) angezeigt, in welchem Zustand bzw. Ausgangszustand sich die Kapsel 20 gerade befindet, d.h. konkret ob die Kapsel 20 an der Wasseroberfläche 31 schwimmt oder ob sie vollständig untergetaucht ist. Der Bediener kann mit Hilfe einer entsprechenden Eingabeeinheit 44, bspw. mit einem Taster oder einem Fußschalter, den in der Software angenommenen und am GUI angezeigten Zustand der Kapsel 20 bei Bedarf manuell ändern. Wird am GUI bspw. angezeigt, dass die Kapsel 20 vollständig untergetaucht ist, der Bediener ist sich aber sicher, dass die Kapsel 20 schwimmt, so kann der Bediener mittels einer Betätigung der Eingabeeinheit 44 den in der Software angenommenen Zustand der Kapsel 20 korrigieren und damit einen geeigneten Anfangszustand für das Bewegungsmodell festlegen.

Die oben beschriebenen Kraftpulse haben entweder ein rampenartiges oder ein kastenförmiges Profil bzw. zeitliches Verhalten. Denkbar sind natürlich auch Kraftpulse mit bspw. einem symmetrischen oder unsymmetrischen dreieckigen Profil oder einem sinus- bzw. cosinus-förmigen Profil. Auch können die Kraftpulse spline-artig, d.h. wie lineare Splines oder Splines höherer Ordnung, geformt sein. Andere Profilformen sind ebenfalls möglich, ein kastenförmiges Profil weist jedoch den Vorteil auf, dass die benötigte Maximalkraft bzw. der benötigte Maximalstrom niedriger ist als bei nichtkastenförmigen Profilen.

Zur Berechnung der einzelnen Kraftpulse mit der Zielsetzung, dass die Kapsel vollständig untertaucht und knapp unter der Oberfläche zum Stillstand kommt, wird der Kraftverlauf zunächst z.B. als Folge von 3 näherungsweise kastenförmigen Kraftpulsen definiert, wobei alle drei Pulse dieselbe zeitliche Länge und Amplitude haben. Es bleiben demnach zwei frei wählbare Parameter, nämlich die Länge und Amplitude des Einzelpulses, zu bestimmen. Diese Bestimmung wird am besten experimentell erfolgen, und zwar in Abhängigkeit von Geometrie und Oberflächenmaterial speziell für diejenige Kapsel, die in einer bestimmten Untersuchung tatsächlich eingesetzt werden soll. Bei der Bestimmung der Parameter wird ggf. auch eine Abhängigkeit von der Kapselausrichtung im Wasser bzw. an der Wasseroberfläche ein Rolle spielen. Der Einfluss des Wassers bspw. abhängig von Temperatur und evtl. Verunreinigungen wird voraussichtlich gering sein, solange keine Zusätze wie bspw. Schäumungsmittel verwendet werden.

Die experimentell bestimmten Kraftverläufe werden in der Software der Ansteuereinheit 42 hinterlegt, z.B. in Form von "Look-up-Tables", die Parameter enthalten, die den Kraftverlauf eindeutig charakterisieren.

## Patentansprüche

1. Vorrichtung zum vollständigen Untertauchen einer an einer Oberfläche (31) einer Flüssigkeit (30) in einem Patienten schwimmenden Endoskopiekapsel (20) in die Flüssigkeit (30) mit Ablösung der Endoskopiekapsel (20) von der Oberfläche (31) der Flüssigkeit (30), mit

- einer Endoskopiekapsel (20), die ein fest integriertes magnetisches Element (21) mit einem magnetischen Dipolmoment ($\vec{m}$) aufweist,
- einem Magnetspulensystem (40) zur Erzeugung eines Magnetfeldes und/oder eines magnetischen Gradientenfeldes zur magnetischen Führung der Endoskopiekapsel (20),
- einer Ansteuereinheit (42) mit hardware- und softwaretechnischen Mitteln zur Steuerung der Bestromung von Einzelspulen (41) des Magnetspulensystems (40),
wobei
- die softwaretechnischen Mittel in der Ansteuereinheit (42) eine Software zur Steuerung der Bestromung der Einzelspulen (41) des Magnetspulensystems (40) enthalten, welche Software das Magnetspulensystem (40) so steuert, dass zur Ablösung der Endoskopiekapsel (20) von der Oberfläche (31) der Flüssigkeit (30) das Magnetspulensystem (40) zur Erzeugung des Magnetfeldes und/oder des magnetischen Gradientenfeldes zur magnetischen Führung der Endoskopiekapsel (20) einen kurzzeitigen Kraftverlauf (F_mag(t)) mit zumindest einem Kraftpuls (F_mag1, F_mag2, F_mag3) auf die Endoskopiekapsel (20) erzeugt, wobei die Richtung des Kraftpulses (F_mag1, F_mag2, F_mag3) im Wesentlichen senkrecht zur Oberfläche (31) der Flüssigkeit (30) steht, wobei der kurzzeitige Kraftverlauf (F_mag(t)) in Abhängigkeit von die Endoskopiekapsel (20) kennzeichnenden Parametern, insbesondere Geometrie, Oberflächenbeschaffenheit und Ausrichtung der Kapsellängsachse relativ zur Wasseroberfläche, sowie von die Flüssigkeit (30) kennzeichnenden Parametern, insbesondere Temperatur, chemische Zusammensetzung, Reinheit und Viskosität, derart vorausberechnet wird, dass die Endoskopiekapsel (20) nach Beendigung der Erzeugung des kurzzeitigen Kraftverlaufs (F_mag(t))
- vollständig untergetaucht ist und
- sich knapp unter der Oberfläche (31) der Flüssigkeit (30) befindet,

und der kurzzeitige Kraftverlauf (F_mag(t)) nach einem Auslösen durch einen Bediener automatisch erzeugt wird.

**2.** Vorrichtung nach Anspruch 1, **dadurch gekennzeichnet, dass** der Kraftpuls (F_mag1, F_mag2, F_mag3) ein weitestgehend kastenförmiges, ein rampenartiges, ein dreickeiges oder ein spline-artiges zeitliches Verhalten (F_mag(t)) aufweist.

**3.** Vorrichtung nach Anspruch 1 oder 2, **dadurch gekennzeichnet, dass** der kurzzeitige Kraftverlauf (F_mag(t)) eine ungerade Anzahl N von Kraftpulsen (F_mag1, F_mag2, F_mag3) mit N>1 umfasst, wobei

- die Richtung der Kraft jedes ungeraden Kraftpulses (F_mag1, F_mag3) in die Flüssigkeit (30) hinein weist, wobei ein zumindest teilweises Eintauchen der Endoskopiekapsel (20) in die Flüssigkeit (30) bewirkt wird, und
- die Richtung der Kraft jedes geraden Kraftpulses (F_mag2) aus der Flüssigkeit (30) heraus weist, wobei ein zumindest teilweises Auftauchen der Endoskopiekapsel (20) aus der Flüssigkeit (30) bewirkt wird.

**4.** Vorrichtung nach Anspruch 1 oder 2, **dadurch gekennzeichnet, dass** der kurzzeitige Kraftverlauf (F_mag(t)) eine gerade Anzahl N von Kraftpulsen (F_mag1, F_mag2) mit N>1 umfasst, wobei

- die Richtung der Kraft jedes ungeraden Kraftpulses (F_mag1) aus der Flüssigkeit (30) heraus weist, wobei ein zumindest teilweises Auftauchen der Endoskopiekapsel (20) aus der Flüssigkeit (30) bewirkt wird, und
- die Richtung der Kraft jedes geraden Kraftpulses (F_mag2) in die Flüssigkeit (30) hinein weist, wobei ein zumindest teilweises Eintauchen der Endoskopiekapsel (20) in die Flüssigkeit (30) bewirkt wird.

**5.** Vorrichtung nach Anspruch 3 oder 4, **dadurch gekennzeichnet, dass** die Beträge der Amplituden der Kraftpulse (|F_mag1|, |F_mag2|, |F_mag3|) und das zeitliche Verhalten der Kraftpulse (F_mag1(t), F_mag2(t), F_mag3(t)) auf eine Schwingresonanz eines Feder-Masse-Systems abgestimmt wird, wobei der Feder-Anteil des Feder-Masse-Systems durch eine Oberflächenspannung der die Endoskopiekapsel (20) umgebenden Flüssigkeit (30) bestimmt wird und der Masse-Anteil aus der Masse (M) der Endoskopiekapsel (20) und der Masse (m) eines die Endoskopiekapsel (20) umgebenden Anteils der Flüssigkeit (30), der mit der Endoskopiekapsel (20) mitbewegt wird, besteht.

**6.** Vorrichtung nach einem der vorhergehenden Ansprüche, **dadurch gekennzeichnet, dass** die Software ein Kapsel-Bewegungsmodell beinhaltet, mit dem ausgehend von einem definierten Ausgangszustand der Endoskopiekapsel (20) und in Abhängigkeit von mittels des Magnetspulensystems (40) auf die Endoskopiekapsel (20) erzeugten magnetischen Kräften ermittelbar ist, ob sich die Endoskopiekapsel (20) gerade an der Oberfläche (31) der Flüssigkeit (30) befindet oder ob die Endoskopiekapsel (20) vollständig untergetaucht ist.

**7.** Vorrichtung nach einem der vorhergehenden Ansprüche, **dadurch gekennzeichnet, dass** der kurzzeitige Kraftverlauf (F_mag(t)) nicht erzeugt werden kann, wenn die Endoskopiekapsel (20) bereits vollständig untergetaucht ist.

**8.** Vorrichtung nach einem der vorhergehenden Ansprüche, **dadurch gekennzeichnet, dass** eine an die Ansteuereinheit (42) angeschlossene Eingabeeinheit (44) vorgesehen ist, mit der ein Bediener manuell vorgeben kann, ob sich die Endoskopiekapsel (20) gerade an der Oberfläche (31) der Flüssigkeit (30) befindet oder ob die Endoskopiekapsel (20) vollständig untergetaucht ist.

**Claims**

**1.** Device for completely immersing an endoscopy capsule (20) floating on a surface (31) of a liquid (30) in a patient into the liquid (30) by separating the endoscopy capsule (20) from the surface (31) of the liquid (30), having

- an endoscopy capsule (20) comprising a fixedly integrated magnetic element (21) with a magnetic dipole moment ($\vec{m}$),
- a solenoid system (40) for generating a magnetic field and/or a magnetic gradient field for magnetic guidance of the endoscopy capsule (20),
- a control unit (42) with technical hardware and software means for controlling the supply of current to individual coils (41) of the solenoid system (40), wherein
- the technical software means in the control unit (42) contain software for controlling the supply of current to the individual coils (41) of the solenoid system (40), which software controls the solenoid system (40) such that to separate the endoscopy capsule (20) from the surface (31) of the liquid (30) the solenoid system (40) for generating the magnetic field and/or the magnetic gradient field for magnetic guidance of the endoscopy capsule (20) generates a brief force curve (F_mag(t)) with at least one force pulse (F_mag1, F_mag2, F_mag3) onto the endoscopy capsule (20), the direction of the force pulse (F_mag1, F_mag2, F_mag3) being substantially perpendicular to the surface (31) of the liquid (30), wherein the brief force curve (F_mag(t)) is calculated in advance, depending on parameters characterising the endoscopy capsule (20), in particular the geometry, surface characteristics and alignment of the capsule longitudinal axis relative to the water surface, and parameters characterising the liquid (30), in particular temperature, chemical composition, clarity and viscosity, such that after the brief force curve (F_mag(t)) has been generated the endoscopy capsule (20)
- is completely immersed and
- is located just below the surface (31) of the liquid (30), and the brief force curve (F_mag(t)) is automatically generated after being triggered by an operator.

2. Device according to claim 1, **characterised in that** the force pulse (F_mag1, F_mag2, F_mag3) has a substantially stepped, ramp-like, triangular or spline-like trend over time (F_mag(t)).

3. Device according to claim 1 or 2, **characterised in that** the brief force curve (F_mag(t)) comprises an odd number N of force pulses (F_mag1, F_mag2, F_mag3) where N>1,

   - the direction of the force of each odd force pulse (F_mag1, F_mag3) facing into the liquid (30), and an at least partial dipping of the endoscopy capsule (20) into the liquid (30) being brought about, and
   - the direction of the force of each even force pulse (F_mag2) facing out of the liquid (30), and an at least partial emersion of the endoscopy capsule (20) from the liquid (30) being brought about.

4. Device according to claim 1 or 2, **characterised in that** the brief force curve (F_mag(t)) has an even number N of force pulses (F_mag1, F_mag2) where N>1,

   - the direction of the force of each odd force pulse (F_mag1) facing out of the liquid (30), and an at least partial emersion of the endoscopy capsule (20) from the liquid (30) being brought about, and
   - the direction of the force of each even force pulse (F_mag2) facing into the liquid (30), and an at least partial dipping of the endoscopy capsule (20) into the liquid (30) being brought about.

5. Device according to claim 3 or 4, **characterised in that** the sizes of the amplitudes of the force pulses (|F_mag1|, |F_mag2|, |F_mag3|) and the trend over time of the force pulses (F_mag1(t), F_mag2(t), F_mag3(t)) are adapted to an oscillation resonance of a spring-mass system, the spring component of the spring-mass system being determined by a surface tension of the liquid (30) surrounding the endoscopy capsule (20), and the mass component consisting of the mass (M) of the endoscopy capsule (20) and the mass (m) of a component of the liquid (30) surrounding the endoscopy capsule (20) which is moved with the endoscopy capsule (20).

6. Device according to one of the preceding claims, **characterised in that** the software contains a capsule-motion model by means of which, assuming a defined initial state of the endoscopy capsule (20) and depending on magnetic forces on the endoscopy capsule (20) generated by means of the solenoid system (40), it may be determined whether the endoscopy capsule (20) is located right on the surface (31) of the liquid (30) or whether the endoscopy capsule (20) is completely immersed.

7. Device according to one of the preceding claims, **characterised in that** the brief force curve (F_mag(t)) cannot be generated when the endoscopy capsule (20) is already completely immersed.

8. Device according to one of the preceding claims, **characterised in that** an input unit (44) connected to the control unit (42) is provided by which an operator is able to predetermine manually whether the endoscopy capsule (20) is located right on the surface (31) of the liquid (30) or whether the endoscopy capsule (20) is completely immersed.

**Revendications**

1. Dispositif pour immerger complètement une capsule (20) endoscopique, flottant sur une surface (31) d'un liquide (30) d'un patient, dans le liquide (30) avec séparation de la capsule (20) endoscopique de la surface (31) du liquide (30), comprenant

   - une capsule (20) endoscopique, qui a un élément (21) magnétique intégré fixement et ayant un moment $(\vec{m})$

dipolaire magnétique,

- un système (40) de bobines excitatrices pour produire un champ magnétique et/ou un champ de gradient magnétique, afin de guider magnétiquement la capsule (20) endoscopique,
- une unité (42) de commande ayant des moyens de la technique matérielle et logicielle pour commander l'alimentation en courant de bobines (41) individuelles du système (40) de bobines excitatrices, dans lequel
- les moyens de la technique logicielle de l'unité (42) de commande comportent un logiciel de commande de l'alimentation en courant de la bobine (41) individuelle du système (40) de bobines excitatrices, lequel logiciel commande le système (40) de bobines excitatrices de manière à ce que, pour séparer la capsule (20) endoscopique de la surface (31) du liquide (30), le système (40) de bobines excitatrices produit, pour produire le champ magnétique et/ou le champ de gradient magnétique, afin de guider magnétiquement la capsule (20) endoscopique, une courbe (F_mag(t)) de force de courte durée ayant au moins une impulsion (F_mag1, F_mag2, F_mag3) de force sur la capsule (20) endoscopique, la direction de l'impulsion (F_mag1, F_mag2, F_mag3 ) de force étant sensiblement perpendiculaire à la surface (31) du liquide (30), la courbe (F_mag(t)) de force de courte durée étant calculée à l'avance en fonction de paramètres caractérisant la capsule (20) endoscopique, notamment la géométrie, l'état de surface et la direction de l'axe longitudinal de la capsule par rapport à la surface de l'eau, ainsi que de paramètres caractérisant le liquide (30), notamment la température, la composition chimique, la pureté, la viscosité, de manière à ce que la capsule (20) endoscopique, après l'achèvement de la production de la courbe (F_mag(t)) de force de courte durée,
- soit immergée complètement et
- se trouve juste en dessous de la surface (31) du liquide (30),

et la courbe (F_mag(t)) de force de courte durée est produite automatiquement par un opérateur après un déclenchement.

2. Dispositif suivant la revendication 1, **caractérisé en ce que** l'impulsion (F_mag1, F_mag2, F_mag3) de force a une courbe (F_mag(t)) dans le temps, dans une grande mesure en forme de caisson, de type à rampe, triangulaire ou de type à spline.

3. Dispositif suivant la revendication 1 ou 2, **caractérisé en ce que** la courbe (F_mag (t)) de force de courte durée comprend un nombre N impair d'impulsions (F_mag1, F_mag2, F_mag3) de force avec N>1, dans lequel

- la direction de la force de chaque impulsion (F_mag1, F_mag2, F_mag3) impaire pénètre dans le liquide (30) en provoquant une immersion au moins partielle de la capsule (20) endoscopique dans le liquide (30), et
- la direction de la force de chaque impulsion (F_mag2) de force paire sort du liquide (30) en provoquant une ascension au moins partielle de la capsule (20) endoscopique hors du liquide (30).

4. Dispositif suivant la revendication 1 ou 2, **caractérisé en ce que** la courbe (F_mag (t)) de force de courte durée comprend un nombre N pair d'impulsions (F_mag1, F_mag2) de force avec N>1, dans lequel

- la direction de la force de chaque impulsion (F_mag1) de force impaire sort du liquide (30) en provoquant, au moins partiellement, une sortie de la capsule (20) endoscopique du liquide (30), et
- la direction de la force de chaque impulsion (F_mag2) de force paire pénètre dans le liquide (30) en provoquant une immersion au moins partielle de la capsule (20) endoscopique dans le liquide (30).

5. Dispositif suivant la revendication 3 ou 4, **caractérisé en ce que** les valeurs absolues des amplitudes des impulsions (|F_mag1|, |F_mag2|, |F_mag3|) de force et la courbe en fonction du temps des impulsions (F_mag1(t), F_mag2(t), F_mag3(t)) de force sont adaptées à un résonnance d'oscillation d'un système ressort-masse, la proportion de ressort du système ressort-masse étant déterminée par une tension superficielle du liquide (30) entourant la capsule (20) endoscopique et la proportion de masse étant constituée de la masse (M) de la capsule (20) endoscopique et de la masse (m) d'une partie du liquide, entourant la capsule (20) endoscopique, qui est entraînée avec la capsule (20) endoscopique.

6. Dispositif suivant l'une des revendications précédentes, **caractérisé en ce que** le logiciel comporte un modèle de déplacement de la capsule, par lequel il peut être calculé, à partir d'un état initial défini de la capsule (20) endoscopique et en fonction de forces magnétiques produites au moyen du système (40) de bobines excitatrices sur la capsule (20) endoscopique, si la capsule (20) endoscopique se trouve juste à la surface (31) du liquide (30) ou si la capsule (20) endoscopique est immergée complètement.

**7.** Dispositif suivant l'une des revendications précédentes, **caractérisé en ce que** la courbe (F_mag(t)) de force de courte durée ne peut pas être produite si la capsule (20) endoscopique est immergée déjà complètement.

**8.** Dispositif suivant l'une des revendications précédentes, **caractérisé en ce qu'**il est prévu une unité (44) d'entrée, qui est raccordée à l'unité (42) de commande et par laquelle un opérateur peut prescrire manuellement que si la capsule (20) endoscopique se trouve juste à la surface (31) du liquide (30) ou que la capsule (20) endoscopique soit immergée complètement.

FIG 1A

FIG 1B

# FIG 2A

F_mag [10<sup>-3</sup>N]; y [cm]

F_mag(t)

y(t)

t [sec]

# FIG 2B

F_mag [10<sup>-3</sup>N]; y [cm]

F_mag(t)

y(t)

t [sec]

EP 2 393 445 B1

FIG 2C

FIG 2D

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102008004871
- US 20070221233 A1
- EP 2143368 A1
- EP 1972253 A1
- DE 10340925 B3